# EUROPEAN PATENT SPECIFICATION

(11) **EP 4 223 233 B1**
(45) Date of publication and mention of the grant of the patent: **09.09.2026**
(21) Application number: 23155384.3
(22) Date of filing: 07.02.2023
(51) Int. Cl.: A61B 17/072, A61B 17/29

(54) **ARTICULATING SURGICAL STAPLING APPARATUS WITH PIVOTABLE KNIFE BAR GUIDE ASSEMBLY**
GELENKIGE CHIRURGISCHE KLAMMERVORRICHTUNG MIT SCHWENKBARER MESSERSTANGENFÜHRUNGSANORDNUNG
APPAREIL D'AGRAFAGE CHIRURGICAL ARTICULÉ AVEC ENSEMBLE DE GUIDAGE DE BARRE DE COUTEAU PIVOTANT

(30) Priority: 07.02.2022 US 202217665962
(43) Date of publication of application: 09.08.2023
(73) Proprietor: Covidien LP, Mansfield, MA 02048 (US)
(72) Inventor: WILLIAMS, Justin P., North Haven, 06473 (US); DELANCY, Griffin S., North Haven, 06473 (US)
(74) Representative: Zimmermann & Partner Patentanwälte mbB

(56) References cited:
- EP-A1- 3 398 528
- EP-A1- 3 718 484
- EP-A1- 3 970 629
- WO-A1-2017/139155
- WO-A2-2016/099959

## Description

### CROSS REFERENCE TO RELATED APPLICATIONS

This application is a continuation-in-part of U.S. Patent Application No. 17/407,628, filed on August 20, 2021.

### TECHNICAL FIELD

This disclosure relates to surgical stapling apparatus, devices and/or systems for performing surgical procedures and methods of use thereof.

### BACKGROUND

Surgical stapling apparatus that clamp, cut and/or staple tissue are well known in the art. Such surgical stapling apparatus include end effectors having two elongated jaw members used to capture or clamp tissue. One of the two jaw members usually carries a staple cartridge that houses a plurality of staples positioned in rows, while the other of the two jaw members has an anvil for forming the staples as the staples are driven from the staple cartridge. For instance, in linear surgical stapling apparatus, a stapling operation is effectuated by a cam bar, a drive sled or other similar mechanism having a cam member that travels longitudinally through channels defined in the staple cartridge and acts upon staple pushers in the channels to sequentially eject linear rows of staples from the staple cartridge. A knife, often supported on a drive beam, is movably positioned between the linear rows of staples such that when the surgical stapling apparatus is positioned about tissue and actuated, the tissue is joined and/or simultaneously or nearly simultaneously cut.

Some surgical stapling apparatus include reloads (sometimes referred to as loading units) and handle assemblies. The reloads may include articulatable end effectors with various articulation angles relative to the handle assemblies. With linear stapling, greater articulation is always desirable with a shorter dead space (e.g., distance from an articulation joint to where a staple line starts). To facilitate articulation, surgical stapling apparatus can include rotatable bar guides to guide knife bars. As the bar guides rotate when the jaw members are articulated, the bar guides can cause the knife bars to distally advance where the greater the articulation, the greater the distal advancement. Advancing of the drive beam requires increased dead space to provide for extra movement and/or require the articulation and drive beam travel to be timed together (e.g., simultaneous two motor movement).

EP 3 718 484 A1 is directed to surgical stapling devices, and more particularly, to surgical stapling devices with tool assemblies that are supported on the stapling device for articulation.

WO 2017/139155 A1 relates to surgical instruments and, in various embodiments, to surgical stapling and cutting instruments and staple cartridges for use therewith.

### SUMMARY

The present invention is defined by the features of the independent claim. Embodiments of the invention are defined in the dependent claims.

This disclosure details surgical stapling apparatus including a bar guide assembly that prevents bar buckling and maintains length during articulation such that a drive beam of the surgical stapling apparatus does not travel upon articulation. Advantageously, the bar guide assembly may be doubled hinged to enable increased articulation and mechanical articulation independent of drive beam travel. Further, the surgical stapling apparatus may include an articulation link with a guide ramp that reduces bend tightness and dead space. In particular, the guide ramp may enable the bar guide assembly to rotate outwardly before bending inwardly to provide a gradual bend. The associated bend path enables the drive beam to be positioned farther back than in stapling apparatus with standard bend paths, thereby providing shorter dead space. This bending movement maintains the drive beam length constant during articulation whereby the drive beam does not move forward during articulation. This bending movement enables articulation and linear drive to be independent of one another, for example, at least up to approximately 50 degrees of articulation relative to a central longitudinal axis of the surgical stapling apparatus. This disclosure also reduces the need for homing the surgical stapling apparatus after a loading unit is secured to the surgical stapling apparatus.

In accordance with one aspect, this disclosure is directed to a surgical stapling apparatus that includes a shaft assembly, an end effector secured to the shaft assembly, a firing assembly, and an articulation assembly. The firing assembly includes a drive beam and a flexible knife bar assembly that are selectively advanceable through the end effector for firing the end effector. The articulation assembly has an articulation link assembly and a pivotable bar guide assembly. The articulation link assembly is coupled to the end effector and actuatable to cause the end effector to articulate relative to the shaft assembly. The pivotable bar guide assembly supports the flexible knife bar assembly and includes a proximal guide assembly and a distal guide assembly that are pivotably coupled together. The proximal guide assembly is pivotably coupled to the shaft assembly. The distal guide assembly is pivotably coupled to the end effector.

In aspects, the pivotable bar guide may pivot in response to an actuation of articulation link assembly. The articulation link assembly may include a proximal articulation link and a distal articulation link that are pivotably coupled together. The distal articulation link may have a proximal end portion coupled to a distal end portion of the proximal articulation link, and a distal end portion coupled to a proximal end portion of the end effector. The proximal articulation link may include a guide ramp positioned to engage the pivotable bar guide when the end effector articulates relative to the shaft assembly.

In aspects, the proximal guide assembly may include a proximal boss that pivotably couples to the shaft assembly. The proximal guide assembly may include a distal hoop defining a boss opening therethrough. The distal guide assembly may include a proximal boss that is received within the boss opening of the distal hoop of the proximal guide assembly to pivotably couple the proximal and distal guide assemblies together. The distal guide assembly may include a distal boss that pivotably couples to a proximal end portion of the end effector. The proximal end portion of the end effector may include a mounting assembly. The mounting assembly may define a boss bore therein that receives the distal boss of the distal guide assembly to pivotably couple the distal guide assembly to the mounting assembly.

In aspects, the pivotable bar guide assembly may define a central passage therethrough that slidably receives the knife bar assembly.

According to another aspect, this disclosure is directed to a surgical stapling apparatus including a housing assembly, an adapter assembly extending from the housing assembly, and a reload selectively attachable to the adapter assembly. The reload supports an end effector on a distal end portion of the reload. The reload includes a firing assembly and an articulation assembly. The firing assembly includes a drive beam and a flexible knife bar assembly that are selectively advanceable through the end effector for firing the end effector. The articulation assembly has an articulation link assembly and a pivotable bar guide assembly. The articulation link assembly is coupled to the end effector and actuatable to cause the end effector to articulate relative to the shaft assembly. The pivotable bar guide assembly supports the flexible knife bar assembly and includes a proximal guide assembly and a distal guide assembly that are pivotably coupled together. The proximal guide assembly is pivotably coupled to the shaft assembly. The distal guide assembly is pivotably coupled to the end effector.

In accordance with yet another aspect, this disclosure is directed to a reload for a surgical stapling apparatus. The reload includes a shaft assembly, an end effector, an articulation link assembly, and a pivotably bar guide assembly. The end effector supports a drive beam and a flexible knife bar assembly that are selectively advanceable through the end effector for firing the end effector. The articulation link assembly is coupled to the end effector and actuatable to cause the end effector to articulate relative to the shaft assembly. The pivotable bar guide assembly is configured to bend the flexible knife bar assembly when the end effector articulates relative to the shaft assembly. The pivotable bar guide assembly includes a proximal guide assembly and a distal guide assembly that are pivotably coupled together. The proximal guide assembly is pivotably coupled to the shaft assembly. The distal guide assembly is pivotably coupled to the end effector.

In accordance with one aspect, this disclosure is directed to a surgical stapling apparatus that includes a shaft assembly, an end effector secured to the shaft assembly, a firing assembly, an articulation assembly, and an articulation guide. The firing assembly includes a flexible knife bar assembly that is selectively advanceable through the end effector for firing the end effector. The articulation assembly has an articulation link assembly and a pivotable bar guide assembly. The articulation link assembly is coupled to the end effector. The articulation link assembly is actuatable to cause the end effector to move relative to the shaft assembly between an unarticulated position and an articulated position. The articulation guide is associated with the pivotable bar guide assembly to enable the flexible knife bar assembly to advance through the pivotable bar guide assembly when the end effector is disposed in the unarticulated position or the articulated position.

In aspects, the pivotable bar guide may pivot in response to an actuation of the articulation link assembly. The articulation link assembly may include a proximal articulation link and a distal articulation link that are pivotably coupled together.

In some aspects, the articulation guide may include a kicker cam, and wherein the kicker cam may extend from the distal articulation link and may engage the pivotable bar guide assembly. The pivotable bar guide assembly may include a proximal guide assembly and a distal guide assembly that are pivotably coupled together. The kicker cam may be configured to slide along an outer surface of the distal guide assembly. The outer surface of the distal guide assembly may include a side bulge, a recess, or combinations thereof that the kicker cam contacts to facilitate articulating movement of the distal guide assembly.

In aspects, the pivotable bar guide assembly may include a proximal guide assembly and a distal guide assembly that are pivotably coupled together. The proximal guide assembly may be pivotably coupled to the shaft assembly. The distal guide assembly may be pivotably coupled to the end effector.

In aspects, the articulation guide may include a detent mechanism associated with the distal guide assembly that facilitates articulating movement of the distal guide assembly.

In aspects, the articulation guide may include a leaf spring that is coupled to the proximal guide assembly to urge the proximal guide assembly to a centered position when articulated.

According to another aspect of this disclosure, a surgical stapling apparatus includes a housing assembly, an adapter assembly extending from the housing assembly, and a reload selectively attachable to the adapter assembly. The reload supports an end effector on a distal end portion of the reload. The reload includes a firing assembly, an articulation assembly, and an articulation guide. The firing assembly includes a flexible knife bar assembly that is selectively advanceable through the end effector for firing the end effector. The articulation assembly has an articulation link assembly and a pivotable bar guide assembly. The articulation link assembly is coupled to the end effector. The articulation link assembly is actuatable to cause the end effector to move relative to the adapter assembly between an unarticulated position and an articulated position. The articulation guide is associated with the pivotable bar guide assembly to enable the flexible knife bar assembly to advance through the pivotable bar guide assembly when the end effector is disposed in the unarticulated position or the articulated position.

In accordance with still another aspect of this disclosure, a reload for a surgical stapling apparatus includes a shaft assembly, an end effector, an articulation link assembly, a pivotable bar guide assembly, and an articulation guide. The end effector supports a flexible knife bar assembly that is selectively advanceable through the end effector for firing the end effector. The articulation link assembly is coupled to the end effector and actuatable to cause the end effector to articulate relative to the shaft assembly between an unarticulated position and an articulated position. The pivotable bar guide assembly is configured to bend the flexible knife bar assembly when the end effector articulates relative to the shaft assembly. The articulation guide is associated with the pivotable bar guide assembly to enable the flexible knife bar assembly to advance through the pivotable bar guide assembly when the end effector is disposed in the unarticulated position or the articulated position.

Other aspects, features, and advantages will be apparent from the description, the drawings, and the claims that follow.

### BRIEF DESCRIPTION OF THE DRAWINGS

The accompanying drawings, which are incorporated in and constitute a part of this specification, illustrate aspects of the disclosure and, together with a general description of the disclosure given above, and the detailed description of the aspect(s) given below, explain the principles of the disclosure, wherein:
FIG. 1 is a perspective view of a surgical stapling apparatus in accordance with the principles of this disclosure;
FIG. 2 is an enlarged, perspective view of the indicated area of detail illustrating an end effector of the surgical stapling apparatus of FIG. 1 in an unarticulated position;
FIG. 3 is another perspective view illustrating the end effector of FIG. 2 in an articulated position;
FIGS. 4-6 are enlarged, perspective views of distal portions of the surgical stapling apparatus of FIG. 1, the distal portions shown with portions thereof removed for clarity;
FIG. 7 is a perspective view, with parts separated, of a distal portion of the surgical stapling apparatus of FIG. 1;
FIG. 8 is a perspective view of a bar guide assembly of the surgical stapling apparatus of FIG. 1;
FIG. 9 is a perspective view, with parts separated, of an upper guide assembly of the bar guide assembly of FIG. 8;
FIG. 10 is a perspective view of a lower guide assembly of the bar guide assembly of FIG. 8;
FIG. 11 is an enlarged, cross-sectional view as taken along section line 11-11 of FIG. 2;
FIG. 12 is a cross-sectional view as taken along section line 12-12 of FIG. 11 with the surgical stapling apparatus shown in the unarticulated position;
FIG. 13 is a view of FIG. 12 illustrating the surgical stapling apparatus in a first articulated position;
FIG. 14 is a view of FIG. 12 illustrating the surgical stapling apparatus in a second articulated position;
FIG. 15 is a perspective view of another distal portion of the surgical stapling apparatus of FIG. 1 in accordance with another aspect, the distal portion shown with portions thereof removed for clarity;
FIGS. 16-20 are top progressive views of a portion of the distal portion of FIG. 15, the views illustrating movement of the distal portion between unarticulated and articulated positions;
FIGS. 21 and 22 are top progressive views of still another distal portion of the surgical stapling apparatus of FIG. 1 in accordance with still another aspect, the views illustrating movement of the distal portion between unarticulated and articulated positions;
FIG. 23 is a top view of yet another distal portion of the surgical stapling apparatus of FIG. 1 in accordance with yet another aspect;
FIG. 24 is a cross-sectional view of FIG. 23 as taken along section line 24-24; and
FIGS. 25 and 26 are top progressive views of the distal portion of FIG. 23, the views illustrating movement of the distal portion between unarticulated and articulated positions.

### DETAILED DESCRIPTION

Aspects of the disclosed surgical stapling apparatus are described in detail with reference to the drawings, in which like reference numerals designate identical or corresponding elements in each of the several views. As commonly known, the term "clinician" refers to a doctor, a nurse, or any other care provider and may include support personnel. Additionally, the term "proximal" refers to the portion of structure that is closer to the clinician and the term "distal" refers to the portion of structure that is farther from the clinician. In addition, directional terms such as front, rear, upper, lower, top, bottom, and the like are used simply for convenience of description and are not intended to limit the disclosure attached hereto. As used herein, the term "endoscopic" is used generally used to refer to endoscopic, laparoscopic, arthroscopic, and/or any other procedure conducted through a small diameter incision or cannula.

As used herein, the terms parallel and perpendicular are understood to include relative configurations that are substantially parallel and substantially perpendicular up to about + or - 10 degrees from true parallel and true perpendicular.

In the following description, well-known functions or constructions are not described in detail to avoid obscuring the present disclosure in unnecessary detail.

Further, although the surgical stapling apparatus are generally shown and described herein in connection with a linear surgical stapling system for brevity, the disclosed surgical stapling apparatus can include any powered, manual, or robotically-controlled surgical stapling systems such as a circular stapler, a transverse stapler, or an open stapler. For a detailed description of the structure and function of exemplary surgical stapling systems, one or more components of which may be included, or modified for use with the disclosed aspects, reference may be made to U.S. Patent Nos. 9,713,470; 8,806,973; 8,256,656; 8,157,152; 8,070,033; 7,819,896; 7,770,774; 7,334,717; 7,128,253; 5,964,394; and 5,915,616.

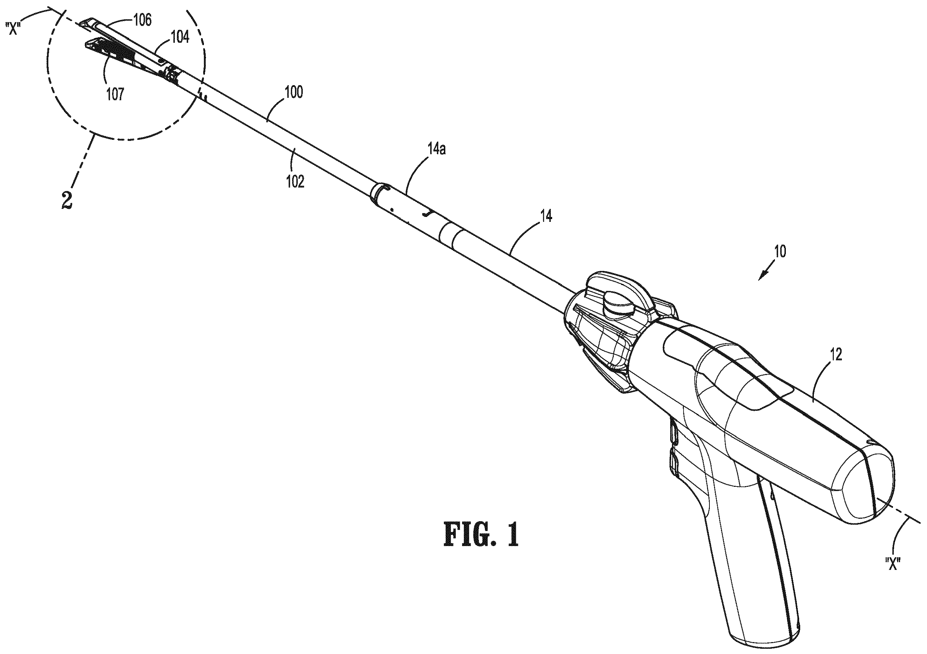

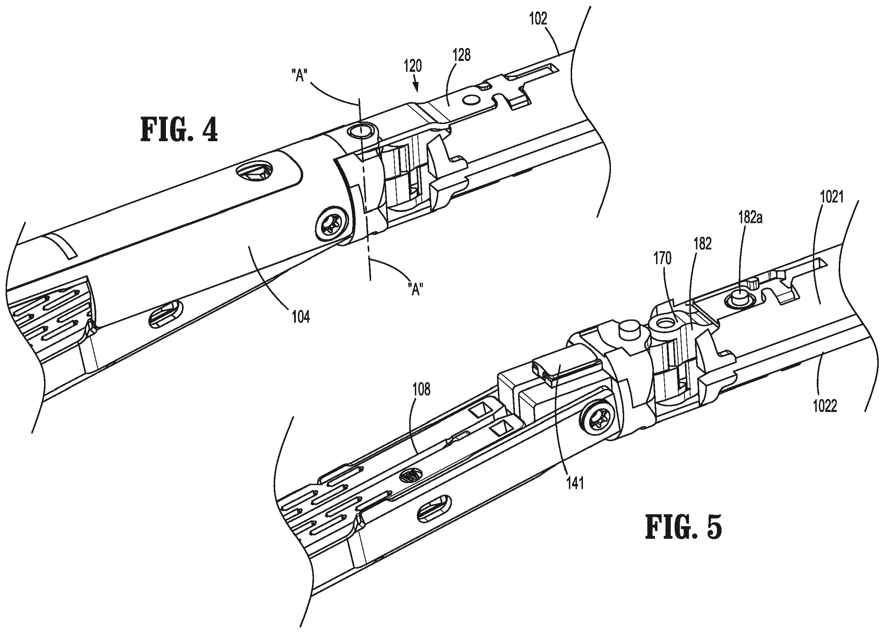

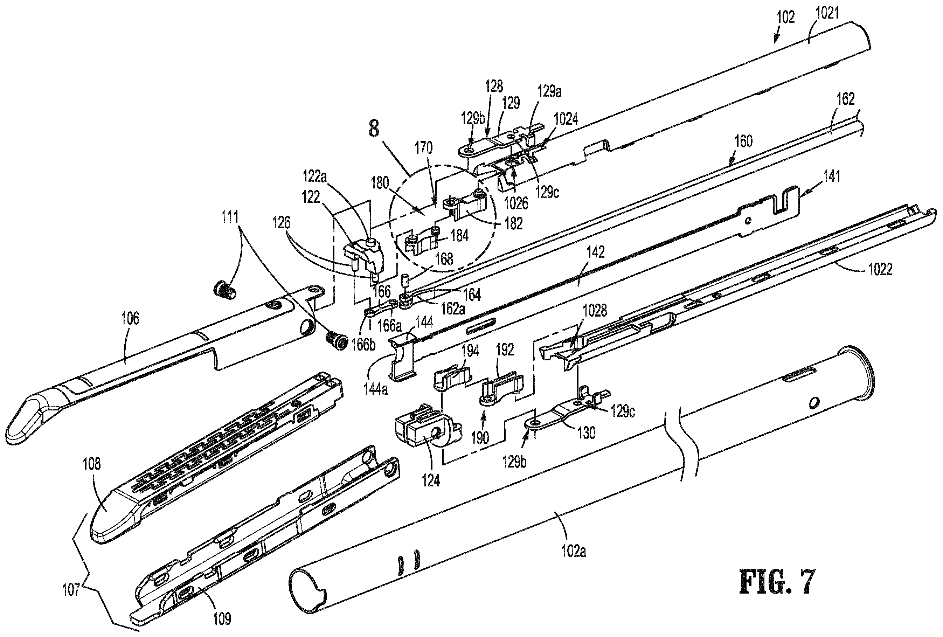

With reference to FIGS. 1-12, a surgical stapling apparatus 10 of this disclosure includes a housing assembly 12 (which may include one or more handles that may be manually actuatable to fire surgical stapling apparatus 10), an adapter assembly 14 secured to housing assembly 12 and extending distally from housing assembly 12, and a reload 100 secured to adapter assembly 14 and extending distally from adapter assembly 14. Adapter assembly 14 and reload 100 define a longitudinal axis "X-X" that extends longitudinally therealong. Reload 100 may be reusable, disposable and/or include one or more reusable and/or disposable components. Housing assembly 12 may be in the form of a powered housing assembly that provides powered actuation of reload 100 via adapter assembly 14.

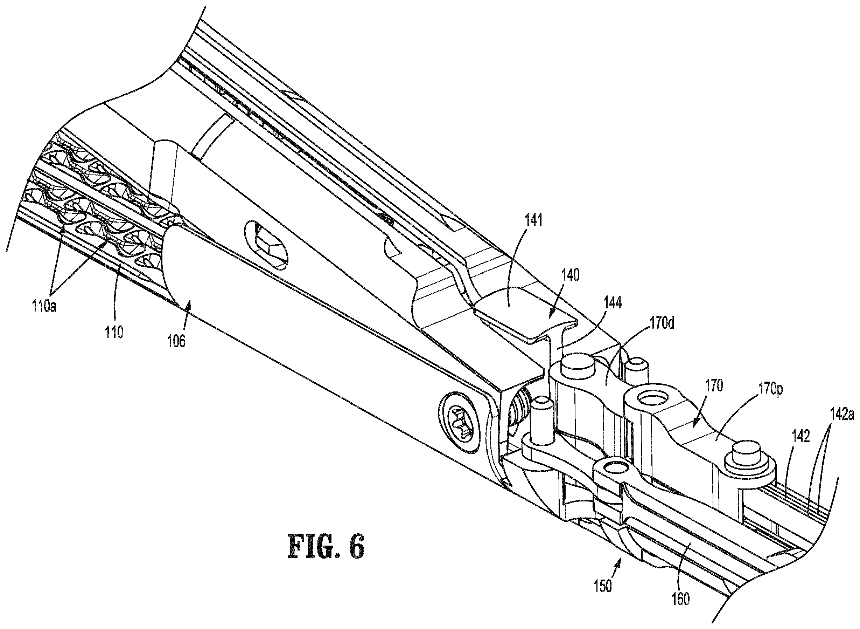

Reload 100 of surgical stapling apparatus 10 is releasably secured to a distal end portion 14a of adapter assembly 14 and includes a shaft assembly 102 that supports an end effector 104 on a distal end portion of shaft assembly 102. End effector 104 includes an anvil assembly 106 and a cartridge assembly 107 that houses a plurality of fasteners or staples in a plurality of rows of staple slots 108a defined in a staple cartridge 108 of cartridge assembly 104. The plurality of rows of staple slots 108a are supported on opposed sides of a knife slot 108c defined through staple cartridge 108. Staple cartridge 108 is selectively attachable to a support plate 109 that is pivotably coupled to anvil assembly 106 via pivot pins 111. Staple cartridge 108 may be selectively removable from support plate 109 and replaceable. Anvil assembly 106 includes an anvil 110 defining a plurality of rows of staple pockets 110a (FIG. 6) therein that correspond with the plurality of rows of staple slots 108a of staple cartridge 108 so that the plurality of staples can be dispensed from staple slots 108a of staple cartridge 108 and formed against staple pockets 110a of anvil 110 upon a firing of surgical stapling apparatus 10.

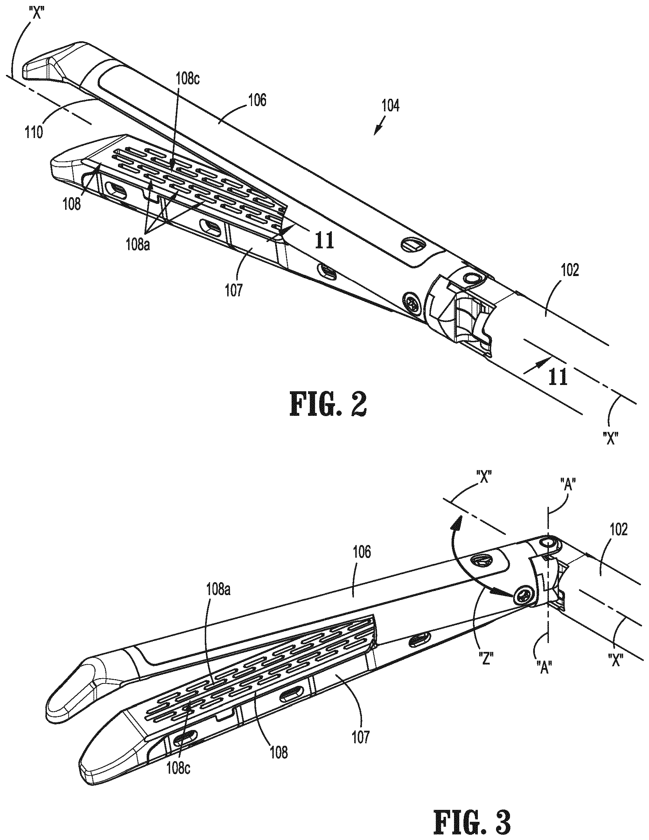

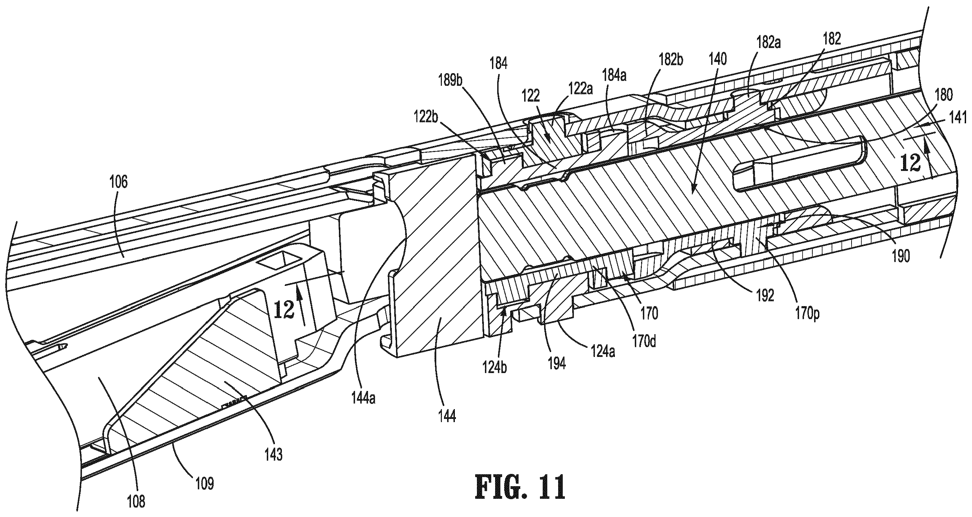

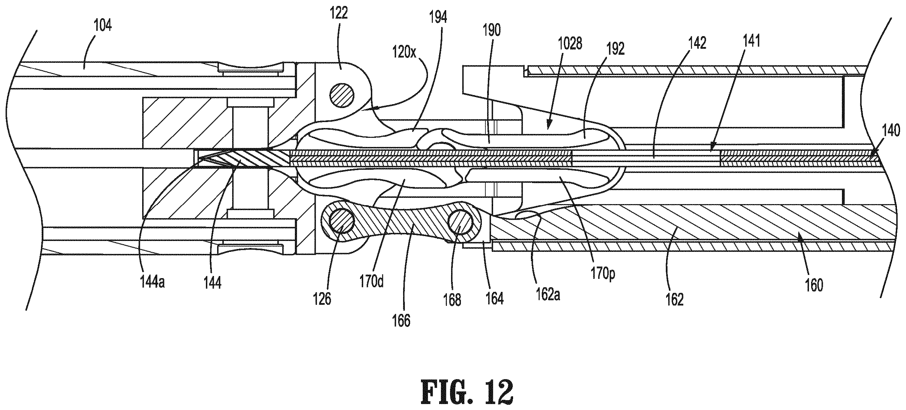

Reload 100 of surgical stapling apparatus 10 supports shaft assembly 102 on a proximal end portion thereof and end effector 104 on a distal end portion thereof. End effector 104 is pivotably coupled to shaft assembly 102 by a mounting assembly 120 that enables end effector 104 to articulate relative to shaft assembly 102 about an articulation axis "A-A" extending through mounting assembly 120, as indicated by arrows "Z." In particular, end effector 104 may pivot about articulation axis "A-A" from an unarticulated position (FIG. 2), aligned with longitudinal axis "X-X" of surgical stapling apparatus 10, to an articulated position (FIG. 3). Mounting assembly 120 includes an upper mounting block 122 and a lower mounting block 124 that are pinned together via mount pins 126 extending from upper mounting block 122. Upper mounting block 122 includes an upper coupler pin 122a extending therefrom and lower mounting block 124 includes a lower coupler pin 124a depending therefrom (see FIG. 11). Upper coupler pin 122a pivotably supports an upper coupling arm 128 and lower coupler pin 124a pivotably supports a lower coupling arm 130. Upper and lower coupling arms 128, 130 extend from mounting assembly 120 and secure mounting assembly 120 to shaft assembly 102. Mounting assembly 120 further defines a proximally-facing concave recess 120x therein (FIG. 12).

Each of upper and lower coupling arms 128, 130 includes an elongated body 129 having a hooked proximal end portion 129a and a distal coupler pin opening 129b defined therethrough. Elongated body 129 further defines a proximal pin opening 129c therethrough.

Shaft assembly 102 of reload 100 includes an outer sleeve 102a and an inner shaft assembly 102b supported within outer sleeve 102a. Inner shaft assembly 102b includes an upper shaft housing 1021 and a lower shaft housing 1022 that couple together and define coupler cutouts 1024 in outer surfaces of distal end portions thereof. Upper and lower shaft housings 1021,1022 have identical structure and are disposed in mirrored relation to one another. Coupler cutouts 1024 of upper and lower shaft housings 1021, 1022 are configured to receive hooked proximal end portions 129a of respective upper and lower coupling arms 128, 130 therein for fixedly securing upper and lower coupling arms 128, 130 to upper and lower shafts housings 1021, 1022. The distal end portions of upper and lower shafts housings 1021, 1022 further define pin apertures 1026 therethrough that are disposed in registration with proximal pin openings 129c of respective upper and lower coupling arms 128, 130. The distal end portion of upper and lower shaft housings 1021, 1022 further define a distally facing concave recess 1028 therein.

Reload 100 of surgical stapling apparatus 10 further includes a firing assembly 140 having a drive beam assembly 141 and sled 143 that is disposed in cartridge assembly 107 for dispensing staples therefrom. Drive beam assembly 141 has a proximal end portion that is operatively coupled to adapter assembly 14 of surgical stapling apparatus 10. Drive beam assembly 141 has a knife bar assembly 142 and a drive beam 144 secured to a distal end portion of knife bar assembly 142 for engagement with sled 143. Knife bar assembly 142 is flexible and includes a plurality of laminates 142a coupled together. Drive beam 144 may be in the form of an I-beam and includes a knife 144a.

Reload 100 further includes an articulation assembly 150 having an articulation link assembly 160 and a pivotable bar guide assembly 170 that is double hinged.

Articulation link assembly 160 of articulation assembly 150 includes a proximal articulation link 162 having a clevis 164 supported on a distal end portion thereof and a guide ramp 162a defined in the distal end portion of articulation link 162 adjacent to a proximal end portion of clevis 164. Articulation link assembly 160 further includes a distal articulation link 166 having a proximal snare 166a and a distal snare 166b. Proximal snare 166a is received by clevis 164 and pinned thereto via link pin 168. Distal snare 166b is coupled to a mount pin 126 of upper mounting block 122.

Pivotable bar guide assembly 170 of articulation assembly 150 includes an upper guide assembly 180 and a lower guide assembly 190 that are coupled together.

Upper guide assembly 180 includes a proximal upper guide 182 and a distal upper guide 184, and lower guide assembly 190 includes a proximal lower guide 192 and a distal lower guide 194. Upper guide assembly 180 and lower guide assembly 190 having mirrored structure such that proximal upper guide 182 and proximal lower guide 192 are identical (but inverted versions of one another) and together form a proximal guide assembly 170p. Distal upper guide 184 and distal lower guide 194 are identical (but inverted versions of one another) and together form a distal guide assembly 170d. Given the symmetry of structure between the upper and lower guide assemblies 180, 190, only upper guide assembly 180 is described hereinbelow for brevity.

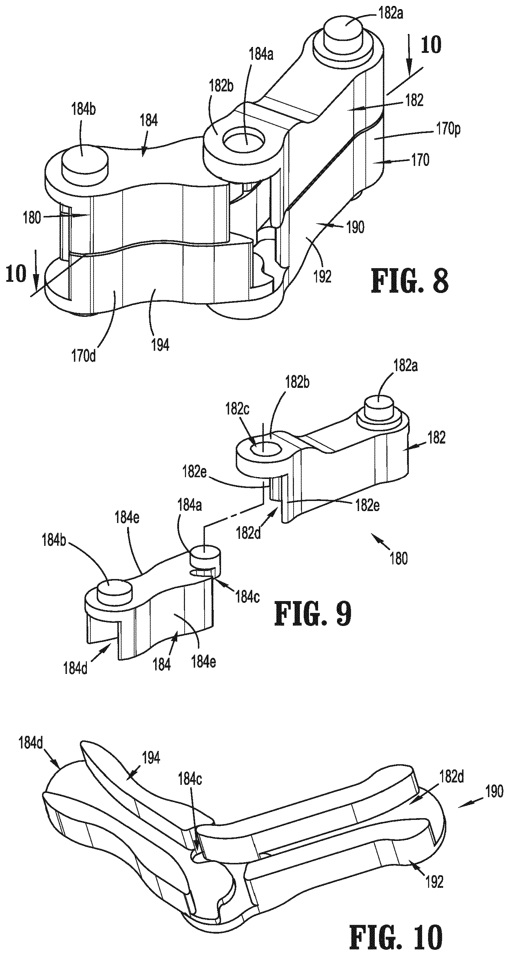

As best seen in FIGS. 8-11, proximal upper guide 182 of upper guide assembly 180 includes a proximal boss 182a projecting from an upper surface thereof and a distal hoop 182b extending distally from proximal upper guide 182. Distal hoop 182b defines a boss opening 182c therethrough. Proximal upper guide 182 further defines a central passage 182d therethrough. Proximal upper guide 182 further includes distal abutments 182e depending from distal hoop 182b.

Distal upper guide 184 of upper guide assembly 180 includes a proximal boss 184a that is received within boss opening 182c of proximal upper guide 182 to pivotably couple distal upper guide 184 and proximal upper guide 182 together. Distal upper guide 184 further includes a distal boss 184b that is received within a boss bore 122b defined within a lower surface of upper mounting block 122 to pivotably couple distal upper guide 184 to upper mounting block 122-notably, a distal boss 184b of distal lower guide 194 of lower guide assembly 190 pivotably couples to a boss bore 124b defined within an upper surface of lower mounting block 124 as seen in FIG. 11. Distal upper guide 184 of upper guide assembly 180 further defines a side slot 184c defined in a proximal portion of distal upper guide 184 adjacent to proximal boss 184a. Distal upper guide 184 also defines a central passage 184d therethrough. Distal upper guide 184 further includes concave sidewalls 184e along a central portion thereof. Central passages 182d, 184d of upper guide assembly 180 are configured to slidably receive knife bar assembly 142 of drive beam assembly 141 therethrough.

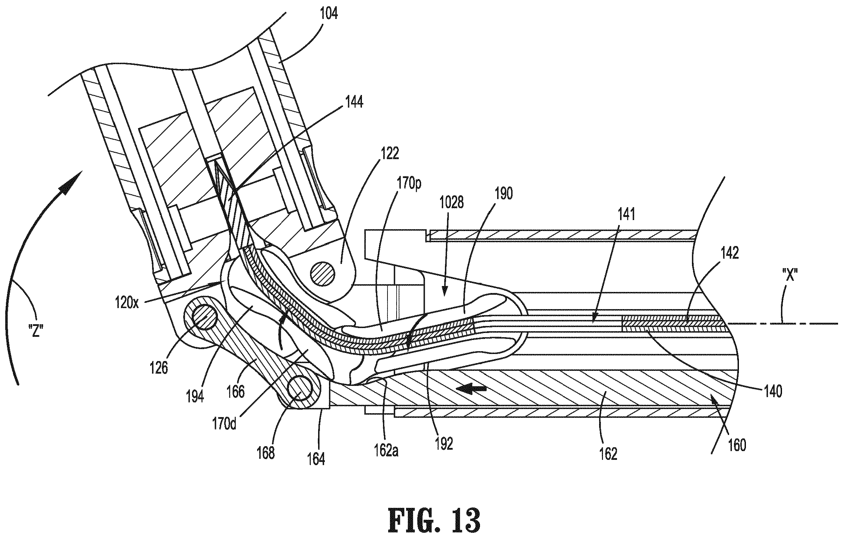

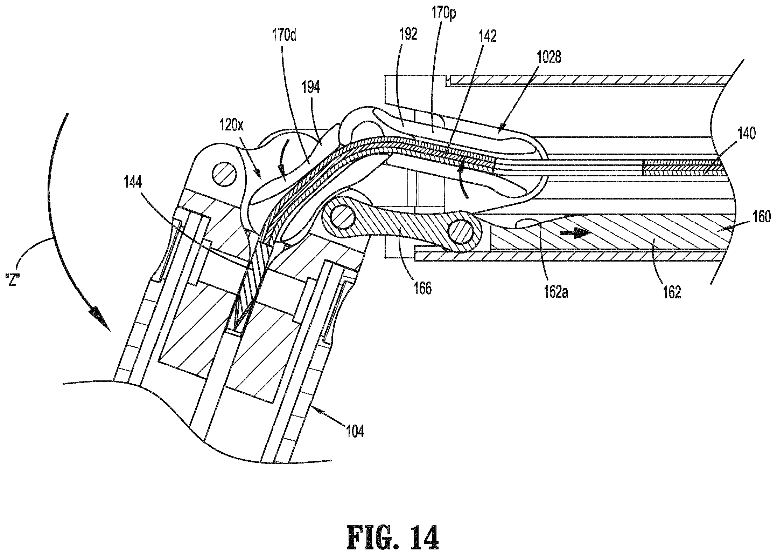

In use, as best seen in FIGS. 2, 3, and 12-14, articulation link assembly 160 can be actuated to distally advance proximal articulation link 162. Distal advancement of proximal articulation link 162 causes distal articulation link 166 to distally advance and pivot about link pin 168 and mount pin 126 to which distal articulation link 166 is coupled. The distal and pivoting movement of distal articulation link 166 articulates end effector 104 in a first direction (e.g., laterally to the right) relative to the longitudinal axis "X" and shaft assembly 102. As end effector 104 articulates in the first direction, proximal guide assembly 170p pivots about proximal bosses 182a in a second direction opposite to the first direction (e.g., laterally to the left) and relative to the longitudinal axis "X." The proximal guide assembly 170p can pivot in the second direction until a distal end portion (e.g., distal hoops 182b) of proximal guide assembly 170p engages guide ramp 162a of proximal articulation link 162. Guide ramp 162a enables pivotable guide bar assembly 170 to cam therealong and facilitate pivotal movement of pivotable guide bar assembly 170. Such pivoting movement causes a proximal end portion of distal guide assembly 170d of pivotable guide bar assembly 170 to pivot with the distal end portion of proximal guide assembly 170p of pivotable guide bar assembly 170 while a distal end portion of distal guide assembly 170d pivots in the first direction with end effector 104. This movement causes pivotable bar guide assembly 170 to bend the portion of knife bar assembly 142 disposed within pivotable bar guide assembly 170 so that upon an actuation of firing assembly 140, knife bar assembly 142 can be distally advanced through end effector 104 when end effector 104 is disposed in such an articulated position to fire surgical stapling apparatus 10.

Articulation link assembly 160 can be actuated to proximally move proximal articulation link 162 so that end effector 104 can be articulated in the second direction. In particular, proximal movement of proximal link 162 of articulation link assembly 160 draws distal articulation link 166 proximally and pivots end effector 104 such that proximal guide assembly 170p pivots in the first direction and distal guide assembly 170d pivots in the second direction to thereby bend the portion of knife bar assembly 142 disposed within pivotable bar guide assembly 170. Again, upon an actuation of firing assembly 140, knife bar assembly 142 can be distally advanced through end effector 104 when end effector 104 is disposed in such an articulated position to fire surgical stapling apparatus 10. Of course, firing assembly 140 can be actuated to advance knife bar assembly 142 distally through the end effector 104 when end effector 104 is in an unarticulated position to fire surgical stapling apparatus 10.

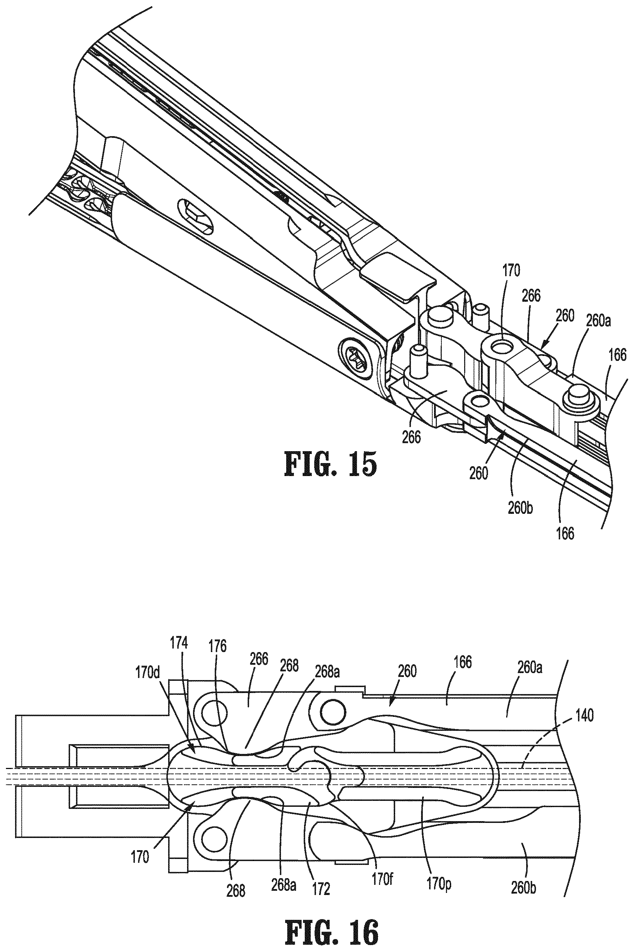

Turning now to FIGS. 15-20, in aspects, articulation assembly 150 includes articulation link assemblies 260 that cooperate with pivotable bar guide assembly 170. Each articulation link assembly 260 includes a proximal articulation link 162 and a distal articulation link 266 that is similar to distal articulation link 166 but includes an articulation guide in the form of a kicker cam 268. Kicker cam 268 has a rounded edge 268a that is disposed in contacting relationship with an outer surface 170f of distal guide assembly 170d of pivotable bar guide assembly 170 to urge distal guide assembly 170 toward a predetermined direction of articulation of end effector 104 as rounded edges 268a of articulation link assemblies 260 cam along outer surface 170f of distal guide assembly 170d.

Outer surface 170f of distal guide assembly 170d includes a proximal side bulge 172, a distal side bulge 174, and a recessed intermediate portion 176 that is disposed between the proximal and distal side bulges 172, 174.

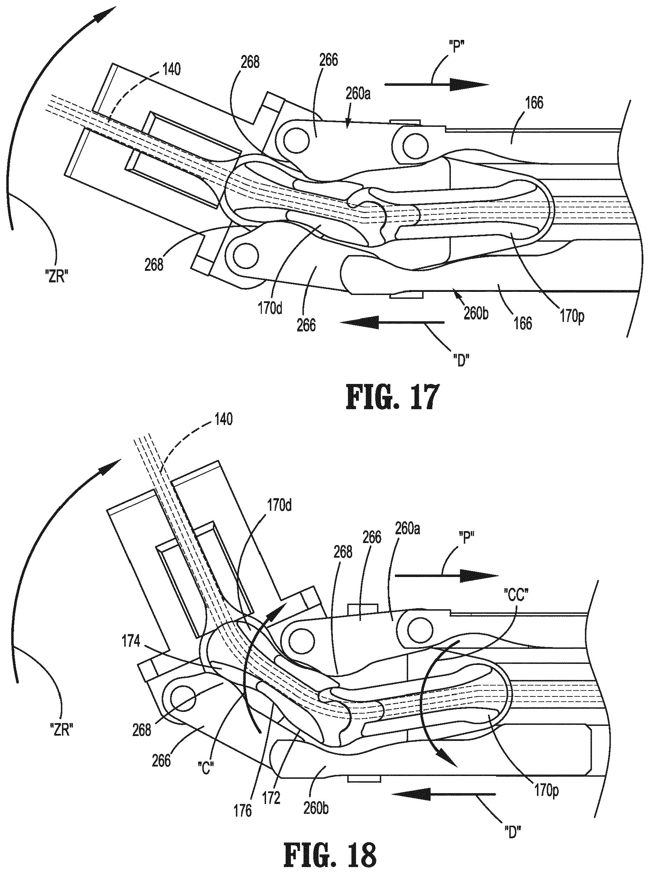

As seen in FIGS. 17 and 18, when a first articulation link assembly 260a of articulation link assemblies 260 is moved in a proximal direction, as indicated by arrow "P," a second articulation link assembly 260b of articulation link assemblies 260 is moved in a distal direction, as indicated by arrow "D," such that articulation link assemblies 260a, 260b urge articulation of end effector 104 in a first direction (e.g., to the right), as indicated by arrow "ZR." In such instance, kicker cam 268 of first articulation link assembly 260a cams proximally out of recessed intermediate portion 176 and proximally along proximal side bulge 172 as kicker cam 268 of second articulation link assembly 260b cams distally out of recessed intermediate portion 176 and distally along distal side bulge 174 causing distal guide assembly 170d to pivot in a clockwise direction, as indicated by arrow "C," as proximal guide assembly 170p pivots in a counterclockwise direction, as indicated by arrow "CC."

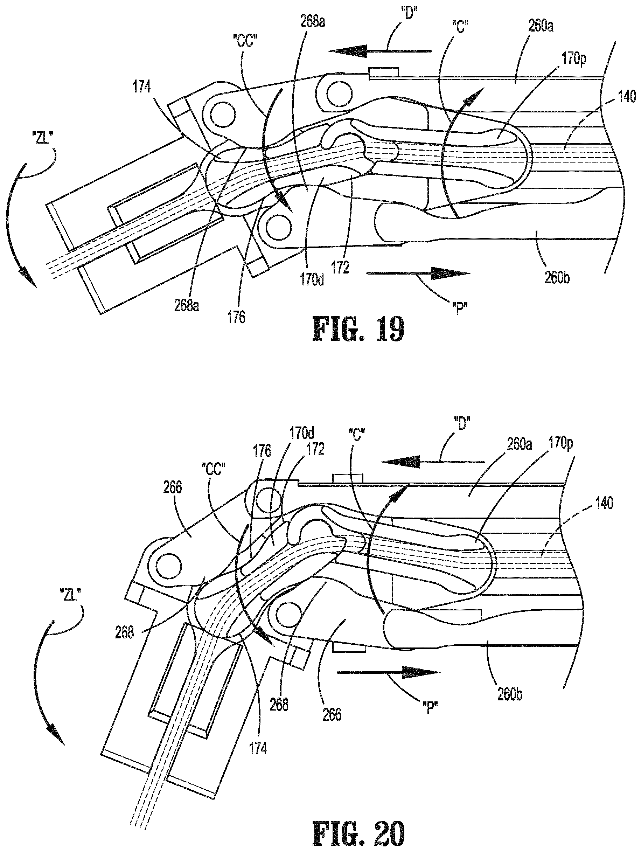

Conversely, as illustrated in FIGS. 19 and 20, when first articulation link assembly 260a is moved in a distal direction, second articulation link assembly 260b moves in a proximal direction such that articulation link assemblies 260a, 260b urge articulation of end effector 104 in a second direction (e.g., to the left) opposite to the first direction, as indicated by arrow "ZL." In such instance, kicker cam 268 of second articulation link assembly 260b cams proximally out of recessed intermediate portion 176 and proximally along proximal side bulge 172 as kicker cam 268 of first articulation link assembly 260a cams distally out of recessed intermediate portion 176 and distally along distal side bulge 174 causing distal guide assembly 170d to pivot in a counterclockwise direction, as indicated by arrow "CC," as proximal guide assembly 170p pivots in a clockwise direction, as indicated by arrow "C."

Notably, the respective distal articulation link 266 of the first or second articulation link assembly 260a, 260b moving in the proximal direction pivots in the same general clockwise or counterclockwise direction as the proximal guide assembly 170p. However, rotation rates of the respective distal articulation link 266 and the proximal guide assembly 170p may be the same and/or different, and may depend on the articulation angle; radii of kicker cams 268, side bulges 172, 174, and/or recessed intermediate portion 176; and/or distances between axes of rotation between articulation link assemblies 260a, 260b and pivotable bar guide assembly 170, or portions thereof. Meanwhile, the respective distal articulation link 266 of the other of the first or second articulation link assemblies 260a, 260b moving in the distal direction pivots in the same general clockwise or counterclockwise direction as the distal guide assembly 170d. However, rotation rates of the respective distal articulation link 266 and the distal guide assembly 170d may be the same and/or different, and may also depend on the articulation angle; radii of kicker cams 268, side bulges 172, 174, and/or recessed intermediate portion 176; and/or distances between axes of rotation between articulation link assemblies 260a, 260b and pivotable bar guide assembly 170, or portions thereof.

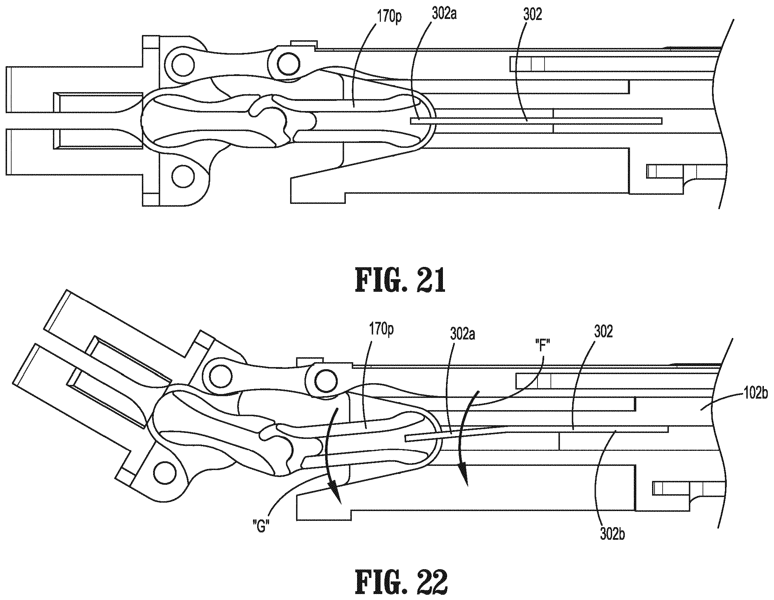

With reference to FIGS. 21 and 22, in one aspect, proximal guide assembly 170p includes an articulation guide in the form of a leaf spring 302 having a distal end portion 302a coupled to a proximal end portion of proximal guide assembly 170p. Leaf spring 302 includes a proximal end portion 302b that is coupled to inner shaft assembly 102b. Distal end portion 302a of leaf spring 302 is configured to flex or articulate, as indicated by arrow "F," relative to proximal end portion 302b of leaf spring 302 as proximal guide assembly 170p pivots or articulates, as indicated by arrow "G." Leaf spring 302 is configured to urge proximal guide assembly 170p toward an initial or unarticulated/unflexed position (FIG. 21) when distal end portion 302a of leaf spring 302 is flexed relative to proximal end portion 302b of leaf spring 302. In aspects, leaf spring 302 may be centered in the unflexed position thereof.

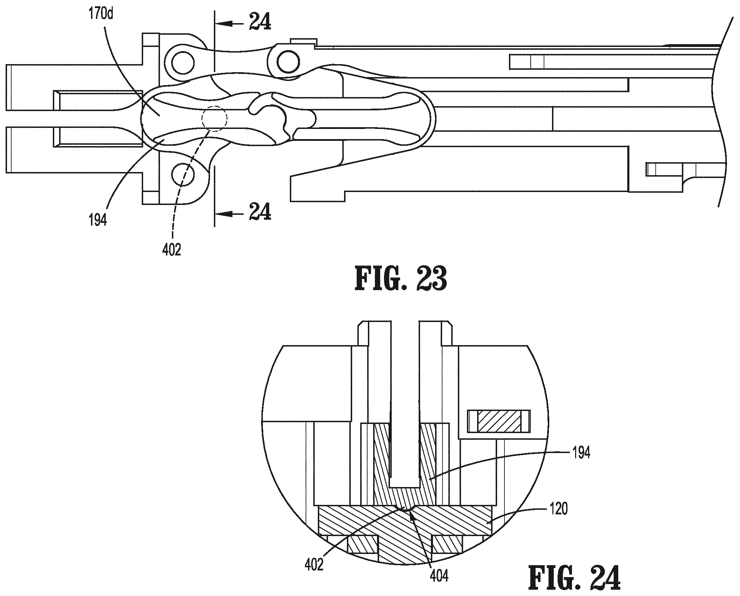

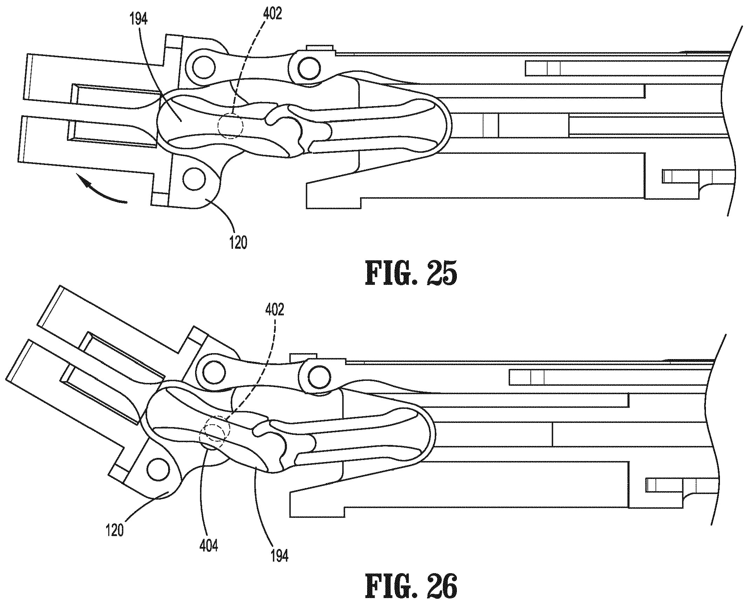

Referring to FIGS. 23-26, in another aspect, articulation guide can be in the form of a detent mechanism 400 including a detent 402 and a detent bore 404. In particular, distal guide assembly 170d, for example, distal lower guide 194 of distal guide assembly 170d, can include detent 402, which may extend from a bottom surface of distal lower guide 194. Mounting assembly 120 may define detent bore 404 therein. Detent bore 404 is configured to selectively receive detent 402 therein. Detent 402 and detent bore 404 are configured to urge distal guide assembly 170d to properly articulate. In aspects, distal guide assembly 170d may defines detent bore 404 and mounting assembly 120 may include detent 402 extending therefrom.

Notably, the bend radius and firing strength requirements of knife bars are highly particular, especially when articulating 25 degrees or more. With such requirements, knife bar set (yielding) can occur (e.g., permanent bends in the laminates 142a of knife bar assembly 142) in response to articulation of end effector 104. Advantageously, the aspects illustrated in FIGS. 15-26 help to prevent pivotable bar guide assembly 170 from inadvertently binding when articulating the knife bar assembly 142 in different directions, particularly when knife bar set occurs. Specifically, these aspects help to center pivotable bar guide assembly 170 and overcome knife bar set so that pivotable bar guide assembly 170 can flex/articulate without binding as the end effector 104 is articulated in either or both directions.

Securement of any of the components of the presently disclosed apparatus may be effectuated using known securement techniques such welding, crimping, gluing, fastening, etc.

The various aspects disclosed herein may also be configured to work with robotic surgical systems and what is commonly referred to as "Telesurgery." Such systems employ various robotic elements to assist the clinician and allow remote operation (or partial remote operation) of surgical instrumentation. Various robotic arms, gears, cams, pulleys, electric and mechanical motors, etc. may be employed for this purpose and may be designed with a robotic surgical system to assist the clinician during an operation or treatment. Such robotic systems may include remotely steerable systems, automatically flexible surgical systems, remotely flexible surgical systems, remotely articulating surgical systems, wireless surgical systems, modular or selectively configurable remotely operated surgical systems, etc.

The robotic surgical systems may be employed with one or more consoles that are next to the operating theater or located in a remote location. In this instance, one team of clinicians may prep the patient for surgery and configure the robotic surgical system with one or more of the instruments disclosed herein while another clinician (or group of clinicians) remotely controls the instruments via the robotic surgical system. As can be appreciated, a highly skilled clinician may perform multiple operations in multiple locations without leaving his/her remote console which can be both economically advantageous and a benefit to the patient or a series of patients. For a detailed description of exemplary medical workstations and/or components thereof, reference may be made to U.S. Patent Application Publication No. 2012/0116416, and PCT Application Publication No. WO2016/025132.

Persons skilled in the art will understand that the structures and methods specifically described herein and shown in the accompanying figures are non-limiting exemplary aspects, and that the description, disclosure, and figures should be construed merely as exemplary of aspects. It is to be understood, therefore, that the present disclosure is not limited to the precise aspects described, and that various other changes and modifications may be effected by one skilled in the art without departing from the scope of the disclosure. Additionally, the elements and features shown or described in connection with certain aspects may be combined with the elements and features of certain other aspects without departing from the scope of the present disclosure, and that such modifications and variations are also included within the scope of the present disclosure. Accordingly, the subject matter of the present disclosure is not limited by what has been particularly shown and described.

## Claims

1. A surgical stapling apparatus (10), comprising:
a shaft assembly (102);
an end effector (104) secured to the shaft assembly;
a firing assembly (140) including a flexible knife bar assembly (142) that is selectively advanceable through the end effector for firing the end effector;
an articulation assembly (150) having an articulation link assembly (160, 260) and a pivotable bar guide assembly (170), wherein the pivotable bar guide assembly includes a proximal guide assembly (170p) and a distal guide assembly (170d) that are pivotably coupled together, the articulation link assembly coupled to the end effector, the articulation link assembly being actuatable to cause the end effector to move relative to the shaft assembly between an unarticulated position and an articulated position; and
an articulation guide (268, 302, 400) associated with the pivotable bar guide assembly to enable the flexible knife bar assembly to advance through the pivotable bar guide assembly when the end effector is disposed in the unarticulated position or the articulated position, wherein the pivotable bar guide pivots in response to an actuation of the articulation link assembly,
wherein the articulation link assembly includes a proximal articulation link (162) and a distal articulation link (166, 266) that are pivotably coupled together, **characterized in that** the articulation guide includes a kicker cam (268), and wherein the kicker cam extends from the distal articulation link and engages the pivotable bar guide assembly.

2. The surgical stapling apparatus of claim 1, wherein the kicker cam is configured to slide along an outer surface (170f) of the distal guide assembly.

3. The surgical stapling apparatus of claim 2, wherein the outer surface of the distal guide assembly includes a side bulge (172, 174), a recess (176), or combinations thereof that the kicker cam contacts to facilitate articulating movement of the distal guide assembly.

4. The surgical stapling apparatus of claim 1, the proximal guide assembly pivotably coupled to the shaft assembly, the distal guide assembly pivotably coupled to the end effector.

5. The surgical stapling apparatus of claim 4, wherein the articulation guide includes a detent mechanism (400) associated with the distal guide assembly that facilitates articulating movement of the distal guide assembly.

6. The surgical stapling apparatus of claim 1, wherein the articulation guide includes a leaf spring (302) that is coupled to the proximal guide assembly to urge the proximal guide assembly to a centered position when articulated.

7. The surgical stapling apparatus of any preceding claim, comprising:
a housing assembly (12);
an adapter assembly (14) extending from the housing assembly; and
a reload (100) selectively attachable to the adapter assembly, the reload supporting the end effector on a distal end portion of the reload, the reload including:
the firing assembly;
the articulation assembly; and
the articulation guide.

8. The surgical stapling apparatus (10) of claim 1, wherein the surgical stapling apparatus is a reload (100).

## Patentansprüche

1. Chirurgische Klammernahtvorrichtung (10), umfassend:
eine Schaftbaugruppe (102),
einen an der Schaftbaugruppe befestigten Endeffektor (104);
eine Auslösebaugruppe (140), die eine flexible Messerschienenbaugruppe (142) aufweist, die gezielt durch den Endeffektor zum Auslösen des Endeffektors vorschiebbar ist;
eine Artikulationsbaugruppe (150) mit einer Artikulationsgliedbaugruppe (160, 260) und einer schwenkbaren Schienenführungsbaugruppe (170), wobei die schwenkbare Schienenführungsbaugruppe eine proximale Führungsbaugruppe (170p) und eine distale Führungsbaugruppe (170d) aufweist, die schwenkbar aneinandergekoppelt sind, wobei die Artikulationsgliedbaugruppe an den Endeffektor gekoppelt ist, wobei die Artikulationsgliedbaugruppe betätigbar ist, um zu bewirken, dass sich der Endeffektor relativ zur Schaftbaugruppe zwischen einer nicht artikulierten Position und einer artikulierten Position bewegt; und
eine Artikulationsführung (268, 302, 400), die mit der schwenkbaren Schienenführungsbaugruppe assoziiert ist, um Vorschieben der flexiblen Messerschienenbaugruppe durch die schwenkbare Schienenführungsbaugruppe zu ermöglichen, wenn der Endeffektor in der nicht artikulierten Position oder in der artikulierten Position angeordnet ist, wobei die schwenkbare Schienenführung als Reaktion auf eine Betätigung der Artikulationsgliedbaugruppe schwenkt,
wobei die Artikulationsgliedbaugruppe ein proximales Artikulationsglied (162) und ein distales Artikulationsglied (166, 266) aufweist, die schwenkbar aneinandergekoppelt sind, **dadurch gekennzeichnet, dass** die Artikulationsführung eine Kicker-Nocke (268) aufweist, und wobei sich die Kicker-Nocke vom distalen Artikulationsglied erstreckt und in die schwenkbare Schienenführungsbaugruppe eingreift.

2. Chirurgische Klammernahtvorrichtung nach Anspruch 1, wobei die Kicker-Nocke zum Gleiten entlang einer Außenfläche (170f) der distalen Führungsbaugruppe ausgelegt ist.

3. Chirurgische Klammernahtvorrichtung nach Anspruch 2, wobei die Außenfläche der distalen Führungsbaugruppe eine seitliche Wölbung (172, 174), eine Vertiefung (176) oder Kombinationen davon aufweist, mit denen die Kicker-Nocke in Kontakt kommt, um eine Artikulationsbewegung der distalen Führungsbaugruppe zu erleichtern.

4. Chirurgische Klammernahtvorrichtung nach Anspruch 1, wobei die proximale Führungsbaugruppe schwenkbar an die Schaftbaugruppe gekoppelt ist und die distale Führungsbaugruppe schwenkbar an den Endeffektor gekoppelt ist.

5. Chirurgische Klammernahtvorrichtung nach Anspruch 4, wobei die Artikulationsführung einen mit der distalen Führungsbaugruppe assoziierten Rastmechanismus (400) aufweist, der eine Artikulationsbewegung der distalen Führungsbaugruppe erleichtert.

6. Chirurgische Klammernahtvorrichtung nach Anspruch 1, wobei die Artikulationsführung eine Blattfeder (302) aufweist, die an die proximale Führungsbaugruppe gekoppelt ist, um die proximale Führungsbaugruppe bei Artikulation in eine zentrierte Position zu drängen.

7. Chirurgische Klammernahtvorrichtung nach einem der vorhergehenden Ansprüche, umfassend:
eine Gehäusebaugruppe (12);
eine sich von der Gehäusebaugruppe erstreckende Adapterbaugruppe (14); und
eine gezielt an der Adapterbaugruppe anbringbare Nachladekassette (100), wobei die Nachladekassette den Endeffektor an einem distalen Endabschnitt der Nachladekassette stützt, wobei die Nachladekassette Folgendes aufweist:
die Auslösebaugruppe;
die Artikulationsbaugruppe; und
die Artikulationsführung.

8. Chirurgische Klammernahtvorrichtung (10) nach Anspruch 1, wobei die chirurgische Klammernahtvorrichtung eine Nachladekassette (100) ist.

## Revendications

1. Appareil d'agrafage chirurgical (10), comprenant :
un ensemble arbre (102) ;
un effecteur terminal (104) fixé à l'ensemble arbre ;
un ensemble de déclenchement (140) comprenant un ensemble barre de couteau flexible (142) qui peut être sélectivement avancé à travers l'effecteur terminal pour déclencher l'effecteur terminal ; et
un ensemble d'articulation (150) comprenant un ensemble de liaison d'articulation (160, 260) et un ensemble de guidage de barre pivotante (170), l'ensemble de guidage de barre pivotante comprenant un ensemble de guidage proximal (170p) et un ensemble de guidage distal (170d) qui sont couplés de manière pivotante ensemble, l'ensemble de liaison d'articulation étant couplé à l'effecteur terminal, l'ensemble de liaison d'articulation étant actionnable pour amener l'effecteur terminal à se déplacer par rapport à l'ensemble arbre entre une position non articulée et une position nonarticulée et une position articulée ; et
un guide d'articulation (268, 302, 400) associé à l'ensemble de guidage de barre pivotante pour permettre à l'ensemble barre de couteau flexible d'avancer à travers l'ensemble de guidage de barre pivotante lorsque l'effecteur terminal est disposé dans la position non articulée ou dans la position articulée, le guide de barre pivotante pivotant en réponse à un actionnement de l'ensemble de liaison d'articulation,
l'ensemble de liaison d'articulation comprenant une liaison d'articulation proximale (162) et une liaison d'articulation distale (166, 266) qui sont couplées de manière pivotante l'une à l'autre, **caractérisé en ce que** le guide d'articulation comprend une came d'éjection (268), et la came d'éjection s'étendant depuis la liaison d'articulation distale et venant en prise avec l'ensemble de guidage de barre pivotante.

2. Appareil d'agrafage chirurgical selon la revendication 1, la came d'éjection étant configurée pour coulisser le long d'une surface extérieure (170f) de l'ensemble de guidage distal.

3. Appareil d'agrafage chirurgical selon la revendication 2, la surface extérieure de l'ensemble de guidage distal comprenant un renflement latéral (172, 174), un évidement (176), ou des combinaisons de ceux-ci avec lequel la came d'éjection entre en contact pour faciliter le mouvement d'articulation de l'ensemble de guidage distal.

4. Appareil d'agrafage chirurgical selon la revendication 1, l'ensemble de guidage proximal étant couplé de manière pivotante à l'ensemble arbre, l'ensemble de guidage distal étant couplé de manière pivotante à l'effecteur terminal.

5. Appareil d'agrafage chirurgical selon la revendication 4, le guide d'articulation comprenant un mécanisme d'encliquetage (400) associé à l'ensemble de guide distal qui facilite le mouvement d'articulation de l'ensemble de guide distal.

6. Appareil d'agrafage chirurgical selon la revendication 1, le guide d'articulation comprenant un ressort à lame (302) qui est couplé à l'ensemble de guide proximal pour pousser l'ensemble de guide proximal vers une position centrée lorsqu'il est articulé.

7. Appareil d'agrafage chirurgical selon l'une quelconque des revendications précédentes, comprenant :
un ensemble boîtier (12) ;
un ensemble adaptateur (14) s'étendant à partir de l'ensemble boîtier ; et
une recharge (100) pouvant être fixée sélectivement à l'ensemble adaptateur, la recharge supportant l'effecteur terminal sur une partie d'extrémité distale de la recharge, la recharge comportant :
l'ensemble de déclenchement ;
l'ensemble d'articulation ; et
le guide d'articulation.

8. Appareil d'agrafage chirurgical (10) selon la revendication 1, l'appareil d'agrafage chirurgical étant une recharge (100).
